(19) Europäisches Patentamt
European Patent Office
Office européen des brevets

(11) Numéro de publication : **0 336 851 B1**

# (12) FASCICULE DE BREVET EUROPEEN

(45) Date de publication du fascicule du brevet : **18.03.92 Bulletin 92/12**

(51) Int. Cl.$^5$ : **A61K 31/66,** A61K 9/20

(21) Numéro de dépôt : **89400964.6**

(22) Date de dépôt : **07.04.89**

## (54) Composition pharmaceutique pour administration orale à base d'un dérivé d'acide diphosphonique.

Le dossier contient des informations techniques présentées postérieurement au dépôt de la demande et ne figurant pas dans le présent fascicule.

(30) Priorité : **07.04.88 FR 8804628**

(43) Date de publication de la demande : **11.10.89 Bulletin 89/41**

(45) Mention de la délivrance du brevet : **18.03.92 Bulletin 92/12**

(84) Etats contractants désignés : **AT BE CH DE FR GB IT LI LU NL SE**

(56) Documents cités :
**FR-A- 2 259 615**
**FR-A- 2 319 645**
**FR-A- 2 319 646**
**FR-A- 2 531 088**
**GB-A- 2 135 879**
**US-A- 4 230 700**

(73) Titulaire : **SANOFI**
**40, Avenue George V**
**F-75008 Paris (FR)**

(72) Inventeur : **Fels, Jean-Pierre**
**3, rue de la Combe Clapiers**
**F-34170 Castelnau le Lez (FR)**
Inventeur : **Gromenil, Jean-Claude**
**6, La voie Romaine**
**F-34560 Montbazin - Poussan (FR)**
Inventeur : **Abramovici, Bernard**
**56, rue du Luminaire**
**F-34990 Juvignac (FR)**

(74) Mandataire : **Gillard, Marie-Louise et al**
**Cabinet Beau de Loménie 55, Rue d'Amsterdam**
**F-75008 Paris (FR)**

**EP 0 336 851 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet une composition pharmaceutique pour l'administration orale à base d'un dérivé d'acide diphosphonique contenant une quantité appropriée de laurylsulfate de sodium. Une telle composition pharmaceutique présente une excellente biodisponibilité chez l'homme.

Dans la description et dans les revendications qui vont suivre, par dérivé d'acide diphosphonique, on entend un composé de formule :

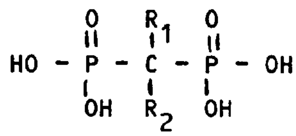

(I)

dans laquelle
$R_1$ représente un atome d'hydrogène, un atome d'halogène, un hydroxyle, un amino, un dialkyl($C_1$-$C_4$)amino ;
$R_2$ représente un atome d'halogène, un alkyle linéaire comportant de 1 à 5 atomes de carbone non substitué ou substitué par un atome de chlore, un hydroxyle, un amino, un dialkyl($C_1$-$C_4$)amino, ou $R_2$ représente un phénoxy, un phényle, un thiol, un phénylthio, un chlorophénylthio, un pyridyle, un thiomorpholinyl-4 ;
et leurs sels avec des acides minéraux ou organiques pharmaceutiquement acceptables.

Ces composés sont connus. Ils ont été décrits comme médicaments dans le traitement par voie orale des affections osseuses ou des affections articulaires du type arthrite, notamment dans les brevets ci-après : BE 902 308, BE 865 434, EP 203 649, DE 2 130 794, BE 822 930, US 4 134 969, EP 162 510, FR 2 525 223, EP 39 033, US 4 578 376, US 4 621 077, JP 55-98193, EP 186 405, EP 100 718, WO 86/00902, WO 87/03598, FR 2 531 088

Parmi ces dérivés d'acides diphosphoniques, on peut citer en particulier les composés suivants :

– l'acide hydroxy-1 éthylidène diphosphonique dont la dénomination commune internationale est l'acide étidronique, et ses sels de sodium ;
– l'acide (pyridinyl-2)-2 éthylidène diphosphonique dont la dénomination commune internationale est l'acide piridronique et ses sels de sodium ;
– l'acide dichlorométhylène diphosphonique dont la dénomination commune internationale est l'acide clodronique et ses sels de sodium ;
– l'acide amino-3 hydroxy-1 propylidène diphosphonique dont la dénomination commune internationale est l'acide pamidronique et ses sels de sodium ;
– l'acide amino-4 hydroxy-1 butylidène diphosphonique et ses sels ;
– l'acide amino-6 hydroxy-1 hexylidène diphosphonique et ses sels ;
– l'acide phénoxy méthylène diphosphonique et ses sels ;
– l'acide thiomorpholino méthylène diphosphonique et ses sels ;
– l'acide chloro-4 phényl thiométhylène diphosphonique, ci-dessous dénommé composé A et ses sels pharmaceutiquement acceptables, notamment le sel disodique du composé A.

Le composé A et ses sels sont décrits dans le brevet français 2 531 088. Ces composés présentent des propriétés antirhumatismales et antiarthritiques, de plus ils inhibent la résorption osseuse. Ces composés peuvent être utilisés dans le traitement de la polyarthrite rhumatoïde, de la maladie de Paget, de l'ostéoporose.

Il est connu que les dérivés de l'acide diphosphonique sont utiles comme médicaments dans le traitement par voie orale des affections osseuses ou des affections articulaires du type arthrite. Il est également connu que ces dérivés sont peu absorbés lorsqu'ils sont administrés par voie orale et que, pour pallier à cette mauvaise absorption, on est conduit à administrer des fortes doses de principe actif.

Le brevet US 4 230 700 décrit une méthode pour le traitement de la maladie de Paget qui comprend l'administration conjointe d'un diphosphonate et d'une vitamine D antirachitique. La description de ce brevet énonce (pages 9, lignes 6 à 10 et lignes 49 - 50 de ce brevet ) :

– que le mode d'administration orale est le mode préféré ;
– que des dosages élevés du principe actif sont obligatoires en raison de l'absorption orale limitée du principe actif (le diphosphonate) ;
– que l'absorption de doses élevées de principe actif produit des effets toxiques et doit être évitée.

L'exemple 1 dudit brevet décrit une gélule ayant la composition suivante :

| | | |
|---|---|---|
| sel disodique de l'acide hydroxy-1 éthylidène diphosphonique | 350 | mg |
| vitamine D3 | 3 000 | U.I |
| amidon | 55,60 | mg |
| laurylsulfate de sodium | 2,90 | mg |

Dans cet exemple, la quantité de laurylsulfate de sodium contenue dans la gélule est d'environ 0,8 % en poids par rapport au sel disodique de l'acide hydroxy-1 éthylidène diphosphonique et d'environ 1 % en poids par rapport à l'acide libre.

La demande de brevet européen 88 462 décrit une composition pharmaceutique comprenant une quantité pharmacologiquement active et efficace d'un organophosphonate et une quantité pharmacologiquement active et efficace d'un stéroïde, ladite composition pouvant aussi contenir des constituants supplémentaires, utilisés de façon conventionnelle dans des compositions pharmaceutiques, dans des proportions habituelles. Le laurylsulfate de sodium est cité parmi ces constituants supplémentaires possibles.

Ce document indique que, par une administration orale, seulement 10 % de l'organophosphonate est absorbé par l'intestin, le reste étant excrété.

Les brevets FR 2 319 645, FR 2 319 646 et FR 2 259 615 décrivent respectivement des acides 1,3-diaminoalcane-1,1-diphosphoniques, des acides 1-hydroxyaminoalcane-1,1-diphosphoniques et des acides aminoalcane diphosphoniques a activité complexante des ions alcalino-terreux, ainsi que les compositions pharmaceutiques ou cosmétiques les contenant. Notamment, ces brevets décrivent une préparation cosmétique pour bain de bouche contenant 4 % en poids de laurylsulfate de sodium par rapport au dérivé d'acide diphosphonique utilisé.

On a maintenant trouvé qu'en ajoutant au dérivé d'acide diphosphonique, dans une composition pharmaceutique orale, une quantité égale ou supérieure à 1,5 % de laurylsulfate de sodium, en poids par rapport au dérivé diphosphonique, on améliore l'absorption dudit dérivé diphosphonique.

On a également trouvé de façon surprenante que cette absorption améliorée se traduit par une augmentation du produit absorbé sans obtention de pics d'absorption pouvant comporter un effet toxique.

De façon tout à fait surprenante, on a trouvé que la meilleure absorption du dérivé d'acide diphosphonique s'accompagne d'une diminution très nette des variations d'absorption entre les individus et ainsi d'une réponse plus homogène de la population traitée vis-à-vis de la dose de dérivé d'acide diphosphonique administrée.

On a finalement trouvé que cette meilleure biodisponibilité permet d'atteindre des taux sanguins suffisants et réguliers avec des doses de dérivé d'acide diphosphonique inférieures aux doses normalement employées en thérapie.

Cette propriété est d'autant plus surprenante si l'on considère que dans les deux documents cités ci-dessus le laurylsulfate de sodium est spécifiquement mentionné, mais soit dans des proportions insuffisantes pour donner un effet potentialisant l'absorption, comme dans le cas du brevet US 4 230 700, soit de façon générique, en tant qu'excipient tensioactif, comme dans le cas du document EP 88 462.

Ainsi, la présente invention a pour objet une composition pharmaceutique à base d'un dérivé d'acide diphosphonique I ou un sel pharmaceutiquement acceptable, caractérisée en ce qu'elle contient, par rapport au dérivé de l'acide diphosphonique, de 1,5 à 6 % en poids de laurylsulfate de sodium.

Selon la présente invention, les compositions pharmaceutiques préférées sont celles contenant un dérivé d'acide diphosphonique choisi parmi l'acide étidronique, l'acide piridronique, l'acide pamidronique, l'acide clodronique, l'acide chloro-4 phényl thiométhylène diphosphonique, l'acide amino-4 hydroxy-1 butylidène diphosphonique, l'acide amino-6 hydroxy-1 hexylidène diphosphonique, l'acide phénoxy méthylène diphosphonique, l'acide thiomorpholino méthylène diphosphonique ou un de leurs sels pharmaceutiquement acceptables.

Une telle composition présente une biodisponibilité améliorée chez l'homme par rapport à une composition ne comprenant pas de laurylsulfate de sodium.

Particulièrement, une telle composition pharmaceutique contient comme principe actif le composé A ou son sel disodique.

Préférentiellement, la composition pharmaceutique comprend entre 100 mg et 500 mg d'acide chloro-4 phényl thiométhylène diphosphonique (composé A) ou l'équivalent en sel disodique et la quantité de laurylsulfate de sodium est comprise entre 1,7 et 4 % en poids par rapport au dérivé d'acide diphosphonique.

La composition pharmaceutique selon l'invention peut être sous forme de comprimé, gélule, poudre, granulé, gouttes ou toute autre forme administrable par voie orale. La forme comprimé est une forme préférée.

La composition selon l'invention peut contenir en outre des ingrédients utilisés habituellement en pharmacie pour la préparation de formes orales. Ainsi la composition selon l'invention peut contenir un agent de désintégration, un agent d'écoulement, un lubrifiant et tout excipient de masse convenable.

Comme excipient de masse, on peut utiliser le lactose, la cellulose ou les amidons. Comme lubrifiant on peut utiliser l'acide stéarique, le stéarate de magnésium, la L-leucine ou, par exemple, le tribéhénate de glycérol. Comme agent de désintégration, on peut utiliser le carboxyméthylamidon sodique, la carboxyméthylcellulose sodique réticulée ou, par exemple, la polyvinylpyrrolidone réticulée. Comme agent d'écoulement, on peut utiliser la silice pure ou le dioxyde de silicium colloïdal.

La présente invention se réfère également aux formes orales à dissolution instantanée et aux formes orales effervescentes obtenues en ajoutant un couple effervescent à la composition selon l'invention. Comme couple effervescent, on peut utiliser, par exemple, l'acide tartrique et le bicarbonate de sodium ou l'acide citrique et le bicarbonate de sodium.

La forme comprimé est une forme préférée selon l'invention. L'invention se réfère également aux comprimés à dissolution instantanée, aux comprimés effervescents ainsi qu'aux comprimés recouverts d'un enrobage.

Les compositions pharmaceutiques sont décrites à titre d'exemples et ne limitent en aucune manière la portée de l'invention.

EXEMPLE 1

Comprimé sécable :

| | |
|---|---|
| - sel disodique du composé A correspondant à 200 mg du composé A | 240 mg |
| - laurylsulfate de sodium | 4,5 mg |
| - carboxyméthylcellulose sodique réticulée | 24 mg |
| - lactose microcristallin | 177 mg |
| - stéarate de magnésium | 4,5 mg |
| | 450 mg |

EXEMPLE 2

Comprimé sécable :

| | |
|---|---|
| - sel disodique du composé A correspondant à 200 mg du composé A | 240 mg |
| - laurylsulfate de sodium | 8 mg |
| - carboxyméthylcellulose sodique réticulée | 24 mg |
| - lactose microcristallin | 173,5 mg |
| - stéarate de magnésium | 4,5 mg |
| | 450 mg |

Une étude de biodisponibilité chez l'homme a été réalisée pour les comprimés préparés selon les exemples 1 et 2.

Pour réaliser cette étude, on a comparé, chez le volontaire sain, les résultats obtenus après administration orale de la composition selon l'exemple 1 de l'invention avec les résultats obtenus après administration orale d'une gélule ou d'un sachet contenant la même quantité de principe actif, correspondant à 200 mg du composé A, mais ne contenant pas de laurylsulfate de sodium.

La gélule contient le principe actif et un excipient approprié. Le sachet contient le principe actif seul.

PROTOCOLE D'ETUDE CLINIQUE

Douze volontaires sains de sexe masculin, âgés de 20 à 28 ans, ont participé à l'étude. Aucun sujet n'avait d'historique médical particulier. Les examens cliniques et de laboratoire étaient normaux. Aucun sujet n'avait reçu de médicament dans les 2 semaines précédant l'étude ni absorbé d'aliment riche en calcium dans les 48 heures immédiatement avant.

Après une nuit de jeûne, les 3 formulations (comprimé, gélule, sachet) ont été absorbées avec 150 ml d'eau minérale à faible teneur en calcium (1,55 mg).

L'étude comporte 3 administrations séparées chacune d'une semaine. Pour chacune des administrations, la dose correspondant à 400 mg de composé A est administrée sous forme de 2 gélules, 2 comprimés ou 2 sachets en une seule prise.

L'ordre d'administration des formulations correspondant à 6 séquences distinctes a été :

| N° du sujet | Ordre d'administration | | |
|---|---|---|---|
| | 1 | 2 | 3 |
| 1,2 | comprimé | gélule | sachet |
| 3,4 | comprimé | sachet | gélule |
| 5,6 | gélule | comprimé | sachet |
| 7,8 | gélule | sachet | comprimé |
| 9,10 | sachet | comprimé | gélule |
| 11,12 | sachet | gélule | comprimé |

Des prélèvements de 8 ml de sang ont été effectués aux temps suivants : avant traitement, puis 0,5 - 1,0 - 1,5 - 2,0 - 2,5 - 3,0 - 4,0 - 6,0 - 8,0 - 10,0 - 12,0 - 24,0 - 36,0 et 48,0 heures après la prise de la composition pharmaceutique. Le principe actif inchangé a été dosé dans ces différents prélèvements.

ANALYSE DES ECHANTILLONS

Le composé A a été dosé par chromatographie liquide haute performance avec détection dans l'UV.

La sélectivité, l'exactitude et la reproductibilité de la méthode ont été vérifiées. La limite de quantification est d'environ 0,05 mg/l.

Une courbe d'étalonnage a été établie chaque jour et des échantillons de contrôle ont été inclus dans chaque cinétique individuelle.

INTERPRETATION PHARMACOCINETIQUE

A partir des concentrations plasmatiques du composé A, les paramètres pharmacocinétiques suivants ont été déterminés :

Cmax : concentration plasmatique maximale observée,
Tmax : délai nécessaire pour atteindre la valeur de Cmax,
SSC : surface sous la courbe des concentrations plasmatiques calculée par la méthode des trapèzes entre les temps 0 et 48 heures.

Afin de faciliter les comparaisons entre les différentes études, les concentrations plasmatiques maximales et les surfaces sous la courbe ont été normalisées à un poids corporel de 70 kg en appliquant le facteur de correction suivant :

$$\text{Paramètre normalisé} = \text{Paramètre brut X } \frac{\text{poids individuel (kg)}}{70}$$

PARAMETRES PHARMACOCINETIQUES

L'examen des profils plasmatiques par sujet révèle une dispersion très forte des résultats pour certains sujets avec les formes sachets et gélules. Ces écarts, sans totalement disparaître, se manifestent à un degré moindre avec la forme comprimé. Les valeurs des Cmax, SSC présentent une variabilité plus faible en ce qui concerne la forme comprimé (coefficient de variation de l'ordre de 30 % pour la forme comprimé et supérieur à 60 % pour les formes gélule et sachet). Les résultats sont rassemblés dans le tableau 1 ci-dessous.

TABLEAU 1

Paramètres pharmacocinétiques moyens obtenus après administration unique de 400 mg du principe actif sous forme de 2 comprimés, de 2 gélules ou de 2 sachets.

| Paramètres | Comprimé | Gélule | Sachet |
|---|---|---|---|
| Tmax (h)<br>(+/- DS) | 1,25<br>(0,40) | 3,15<br>(0,87) | 3,13<br>(1,03) |
| Cmax* (mg/l)<br>(+/- DS) | 3,00<br>(0,98) | 1,06<br>(0,92) | 0,97<br>(0,62) |
| SSC 0-48 h*<br>(mg.h/l)<br>(+/- DS) | 30,91<br>(9,79) | 11,94<br>(7,63) | 12,48<br>(7,75) |

Les déviations standards (+/- DS) sont indiquées pour chaque valeurs.

* Paramètre normalisé pour un poids corporel de 70 kg.

Le comprimé conduit à des délais d'obtention du pic des concentrations plasmatiques (Tmax) significativement plus courts qu'avec la gélule et le sachet (respectivement pour le comprimé 1,25 +/- 0,40 h, pour la gélule 3,15 +/- 0,87 h et pour le sachet 3,13 +/- 1,03 h). La différence sachet contre gélule n'est pas statistiquement significative.

Le comprimé conduit à des concentrations plasmatiques maximales (Cmax) nettement plus élevées avec des différences très hautement significatives aussi bien vis-à-vis de la gélule que vis-à-vis du sachet (respectivement pour le comprimé 3,00 +/-0,98 mg/l, pour la gélule 1,06 +/- 0,92 mg/l et pour le sachet 0,97 +/- 0,62 mg/l). La différence sachet contre gélule n'est pas statistiquement significative.

La comparaison des surfaces sous la courbe (SSC) à 48 heures des concentrations plasmatiques pour les 3 formes étudiées met en évidence la biodisponibilité en moyenne 3 fois supérieure du comprimé selon l'invention par rapport aux deux autres formes. Les différences entre les surfaces sous les courbes sont très hautement significatives entre le comprimé et le sachet ou le comprimé et la gélule. Par contre, on ne note pas d'écart significatif entre la gélule et le sachet.

L'étude de biodisponibilité chez l'homme a été reprise dans les mêmes conditions avec des comprimés préparés selon l'exemple 2.

Les résultats obtenus sont rassemblés ci-après :

- Tmax : 1,56 h (+/- 1,02)

- Cmax* : 2,46 mg/l (+/- 0,98)
- SSC* 0-48 heures : 23,94 mg.h/l (+/- 5,21).

Les déviations standards (+/- DS) sont indiquées.

* Paramètre normalisé pour un poids corporel de 70 hg.

Ces résultats sont comparables à ceux obtenus avec les comprimés préparés selon l'exemple 1.

CONCLUSION DE L'ETUDE

La composition pharmaceutique selon l'invention permet d'obtenir :

– une absorption plus rapide du composé A,

– une biodisponibilité environ 3 fois supérieure par rapport aux deux autres formes,

– une variabilité interindividuelle réduite au niveau des concentrations plasmatiques.

Aucun signe de mauvaise tolérance n'a été observé après administration de la composition pharmaceutique selon l'invention.

## Revendications

1. Composition pharmaceutique pour administration orale à base d'un dérivé d'acide diphosphonique de formule :

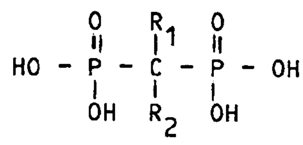

(I)

dans laquelle
$R_1$ représente un atome d'hydrogène, un atome d'halogène, un hydroxyle, un amino, un dialkyl($C_1$-$C_4$)amino ;
$R_2$ représente un atome d'halogène, un alkyle linéaire comportant de 1 à 5 atomes de carbone non substitué ou substitué par un atome de chlore, un hydroxyle, un amino, un dialkyl($C_1$-$C_4$)amino, ou $R_2$ représente un phénoxy, un phényle, un thiol, un phénylthio, un chlorophénylthio, un pyridyle, un thiomorpholinyl-4 ;
ou un de ses sels pharmaceutiquement acceptables, caractérisé en ce qu'elle contient, par rapport au dérivé de l'acide diphosphonique, de 1,5 à 6 % en poids de laurylsulfate de sodium.

2. Composition pharmaceutique selon la revendication 1, caractérisé en ce que le dérivé d'acide diphosphonique est choisi parmi l'acide étidronique, l'acide piridronique, l'acide clodronique, l'acide pamidronique, l'acide chloro-4 phényl thiométhylène diphosphonique, l'acide amino-4 hydroxy-1 butylène diphosphonique, l'acide amino-6 hydroxy-1 hexylidène diphosphonique, l'acide phénoxy méthylène diphosphonique, l'acide thiomorpholino méthylène diphosphonique ou un de leurs sels pharmaceutiquement acceptables.

3. Composition pharmaceutique selon la revendication 1, dans laquelle le dérivé d'acide diphosphonique est l'acide chloro-4 phényl thiométhylène diphosphonique ou son sel disodique.

4. Composition pharmaceutique selon l'une quelconque des revendications 1, 2 ou 3, comprenant de 100 à 500 mg d'acide chloro-4 phényl thiométhylène diphosphonique ou d'équivalent en sel disodique et de 1,7 à 4 % en poids de laurylsulfate de sodium.

## Claims

1. Pharmaceutical composition for oral administration, based on a diphosphonic acid derivative of the formula

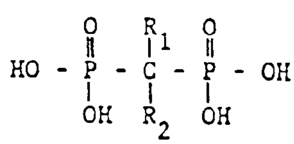

(I)

in which

$R_1$ represents a hydrogen atom, a halogen atom, a hydroxyl, an amino or a dialkyly($C_1$-$C_4$)amino;
$R_2$ represents a halogen atom, a linear alkyl containing from 1 to 5 carbon atoms which is unsubstituted or substituted by a chlorine atom, a hydroxyl, an amino or a dialkly($C_1$-$C_4$)amino, or $R_2$ represents a phenoxy, a phenyl, a thiol, a phenylthio, a chlorophenylthio, a pyridyl or a thiomorpholin-4-yl,
or one of its pharmaceutically acceptable salts, characterized in that it comprises from 1.5 to 6% by weight of sodium laurylsulfate relative to the diphosphonic acid derivative.

2. Pharmaceutical composition according to claim 1, characterized in that the diphosphonic acid derivative is selected from etidronic acid, piridronic acid, clodronic acid, pamidronic acid, 4-chlorophenylthiomethylenediphosphonic acid, 4-amino-1-hydroxybutylenediphosphonic acid, 6-amino-1-hydroxyhexylidenedi phosphonic acid, phenoxymethylenediphosphonic acid, thiomorpholinomethylenediphosphonic acid or one of their pharmaceutically acceptable salts.

3. Pharmaceutical composition according to claim 1, in which the diphosphonic acid derivative is 4-chlorophenylthiomethylenediphosphonic acid or its disodium salt.

4. Pharmaceutical composition according to any one of claims 1, 2 or 3, comprising from 100 to 500 mg of 4-chlorophenylthiomethylenediphosphonic acid or the equivalent amount of its disodium salt, and from 1.7 to 4% by weight of sodium laurylsulfate.

## Patentansprüche

1. Pharmazeutische Zusammensetzung zur oralen Verabreichung auf Basis eines Diphosphonsäurederivats der Formel

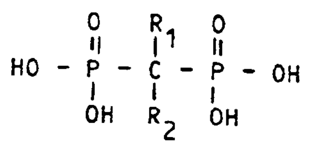

(I)

worin
$R_1$ ein Wasserstoffatom, ein Halogenatom, ein Hydroxyl, ein Amin oder ein Dialkyl($C_1$-$C_4$)-amin darstellt;
$R_2$ ein Halogenatom, ein nicht substituiertes oder durch ein Chloratom, Hydroxyl, Amin, Dialkyl($C_1$-$C_4$)-amin substituiertes gerades Alkyl mit 1 bis 5 Kohlenstoffatomen darstellt, oder $R_2$ für Phenoxy, Phenyl, Thiol, Phenylthio, Chlorphenylthio, Pyridyl, 4-Thiomorpholinyl steht;
oder eines seiner pharmazeutisch akzeptablen Salze, dadurch gekennzeichnet, daß sie, bezogen auf das Diphosphonsäurederivat, 1,5 bis 6 Gew.% Natriumlaurylsulfat enthält.

2. Pharmazeutische Zusammensetzung nach Anspruch 1, dadurch gekennzeichnet, daß das Diphosphonsäurederivat ausgewählt ist aus Etidronsäure, Piridronsäure, Clodronsäure, Pamidronsäure, 4-Chlorphenylthiomethylendiphosphonsäure, 4-Amino-1-hydroxy-butylen-diphosphonsäure, 6-Amino-1-hydroxy-hexyliden-diphosphonsäure, Phenoxymethylen-diphosphonsäure, Thiomorpholinmethylendiphosphonsäure oder einem ihrer pharmazeutisch akzeptablen Salze.

3. Pharmazeutische Zusammensetzung nach Anspruch 1, bei welcher das Diphosphonsäurederivat 4-Chlorphenylthiomethylen-diphosphonsäure oder ihr Dinatriumsalz ist.

4. Pharmazeutische Zusammensetzung nach einem der Ansprüche 1, 2 oder 3, umfassend 100 bis 500 mg 4-Chlorphenyl-thiomethylen-diphosphonsäure oder das Äquivalent an Dinatriumsalz und 1,7 bis 4 Gew.% Natriumlaurylsulfat.